# EUROPEAN PATENT SPECIFICATION

(11) **EP 2 897 478 B1**
(45) Date of publication and mention of the grant of the patent: **07.11.2018**
(21) Application number: 13766638.4
(22) Date of filing: 18.09.2013
(51) Int. Cl.: A24F 47/00, A61M 15/06, A61M 11/04

(54) **HEATING SMOKEABLE MATERIAL**
ERWÄRMUNG VON RAUCHBAREM MATERIAL
CHAUFFAGE D'UN MATÉRIAU À FUMER

(30) Priority: 18.09.2012 GB 201216621
(43) Date of publication of application: 29.07.2015
(73) Proprietor: British American Tobacco (Investments) Ltd, London, WC2R 3LA (GB)
(72) Inventor: PHILLIPS, Jeremy, London WC2R 3LA (GB); WOODMAN, Thomas, London WC2R 3LA (GB); BITAR, Ahmed, Hertfordshire SG8 7AJ (GB); BRERETON, Simon, Hertfordshire SG7 5PR (GB); FARENDEN, Paul, Hertfordshire SG9 9HP (GB); HATRICK, David, Therwil 4106 (CH); McGINLEY, Ryan, Cambridgeshire CB23 6FP (GB); TWELFTREE, Tom, Oxfordshire OX9 2EE (GB); VASISHTA, Viju, London SW1W 9SR (GB); WEST, Grant, Bedfordshire LU2 9QA (GB)
(74) Representative: EIP
(86) International application number: PCT/GB2013/052433
(87) International publication number: WO 2014/045025

(56) References cited:
- EP-A1- 1 609 376
- EP-A1- 2 138 058
- DE-A1- 19 854 009
- US-A1- 2006 118 128

## Description

### Field

The invention relates to heating smokeable material in order to volatilise components of the smokeable material.

### Background

Smoking articles such as cigarettes and cigars burn tobacco during use to create tobacco smoke. Attempts have been made to provide alternatives to these smoking articles by creating products which release compounds without creating tobacco smoke. Examples of such products are so-called heat-not-burn products which release compounds by heating, but not burning, tobacco.

EP 2 138 058 A discloses an apparatus according to the preamble of claim 1.

### Summary

According to a first aspect of the invention, there is provided the apparatus of claim 1.
In some embodiments, the apparatus further comprises smokeable material to be heated so that at least one component of said smokeable material is volatilised.
In some embodiments, the heat source and/or the smokeable material is heated to a target temperature of 80-125°C. In other embodiments, the heat source and/or the smokeable material is heated to a target temperature of 40-80°C.
In some embodiments, the target temperature is reached within no more than 5 minutes from actuation of the actuating mechanism.
In some embodiments, the target temperature is maintained for a period of at least 3 minutes.
Optionally, the phase change material is selected from the group consisting of: sodium acetate trihydrate, sodium hydroxide monohydrate, barium hydroxide octahydrate, magnesium nitrate hexahydrate and magnesium chloride hexahydrate.

In some embodiments, at least a portion of the housing is clear or translucent and/or at least a portion of the wall of the heat source chamber is clear or translucent.

In some embodiments, the heat source chamber at least partially surrounds the heating chamber.

### Brief Description of the Figures

For the purposes of example only, embodiments of the invention are described below with reference to the accompanying drawings, in which:
Figure 1 is an illustration of a cross-sectional view of an apparatus configured to heat smokeable material to release aromatic compounds and/or nicotine from the smokeable material;
Figure 2 is an exploded view of another apparatus;
Figure 3 is a graph showing the heat generated by different reagents combined with water to cause an exothermic reaction;
Figure 4 is a graph showing the heat generated by combining magnesium sulphate with different volumes of water;
Figures 5a and 5b are graphs showing the heat generated using different forms of calcium chloride with varying volumes of water;
Figures 6a, 6b and 6c are graphs showing the heat generated using different forms of sodium hydroxide with varying volumes of water;
Figures 7a, 7b and 7c are graphs showing the heat generated by calcium oxide powder and/or chunks with different volumes of water and in different reaction vessels;
Figure 8a and 8b are graphs showing the temperatures of different parts of the apparatus when the apparatus is held in vertical and horizontal orientations respectively;
Figure 9 is a graph showing the effect of air flow through the device on the temperature of the heat source and of the smokeable material;
Figure 10 is an illustration of a cross-sectional view of another apparatus configured to heat smokeable material to release aromatic compounds and/or nicotine from the smokeable material;
Figure 11 is a graph showing the heat generated by the phase change of sodium acetate trihydrate; and
Figure 12 is a graph showing the effect of air flow through the device on the temperature within the apparatus and of the surface of the apparatus.

### Detailed Description

As used herein, the term 'smokeable material' includes any material that provides volatilised components upon heating and includes any tobacco-containing material and may, for example, include one or more of tobacco, tobacco derivatives, treated or modified tobacco such as expanded tobacco and reconstituted tobacco, or tobacco substitutes.

In some embodiments, the smokeable material provides enhanced release of volatile components at temperatures below their boiling point. This may be achieved by using temperature to increase the vapour pressure of a substance.

According to the invention, there is provided an apparatus configured to heat smokeable material using a chemical heat source to volatilise at least one component of the smokeable material. The apparatus may be configured to heat the smokeable material without combusting the smokeable material. In some embodiments, the apparatus is a smokeless inhalation device, for example delivering nicotine and optionally other components such as flavours and aromas, in an inhalable form. In some embodiments, the apparatus is an aerosol generating device. Such devices produce an inhalable aerosol which, in some embodiments, may contain nicotine.

In some embodiments, the chemical heat source generates sufficient heat to heat the smokeable material and to volatilise at least one component within a period of no more than about 5 minutes from actuation of the actuating mechanism, no more than 4 minutes, no more than 3 minutes, no more than 2 minutes, no more than 1 minute, no more than 45 seconds, no more than 30 seconds, no more than 25 seconds, no more than 20 seconds, no more than 15 seconds or no more than 10 seconds. In some embodiments, the period from actuation of the device to volatilisation of at least one component is from about 10 to about 60 seconds (under standard conditions, i.e. at temperatures and pressures to which devices of this nature would normally be expected to be exposed during use by a consumer).

In some embodiments, the heat source generates sufficient heat to heat the smokeable material and to maintain a temperature within a target range, so as to volatilise at least one component for a period of at least about 3 minutes from actuation of the actuating mechanism, and in some embodiments, a period of at least about 5 minutes.

In some embodiments, the apparatus will include sufficient smokeable material to provide a user with at least 4 puffs and, in some embodiments, with about 7 to 8 puffs. In some embodiments, the apparatus is capable of providing sufficient vapour for a 35ml puff every 60 seconds. In some embodiments, the apparatus is capable of providing sufficient vapour for a 55ml puff every 30 seconds. In some embodiments, the total nicotine in the vapour generated by the apparatus is from about 0.01 to about 0.5 mg, and in some embodiments is from about 0.05 to about 0.1 mg.

In some examples, the heat provided by the heat source can be produced by an exothermic chemical reaction between two or more reagents. This type of heat source may provide a relatively high temperature. As such, an apparatus having a heat source which involves an exothermic chemical reaction between two or more reagents is referred to herein as a high temperature device.

In some embodiments, the heat source is capable of heating at least a portion of the smokeable material to a temperature of about 60-125°C. In some embodiments, the heat source is heated to a temperature of about 60-125°C upon actuation. In further embodiments, the temperature of the heat source and/or of at least a portion the heated smokeable material is about 60-100°C, 65-95°C, 70-90°C, 75-85°C or about 80°C. In some embodiments, the target temperature of the heated smokeable material is from about 80°C +/- 10°C, i.e. about 70 to about 90°C.

Alternatively or in addition, the heat source may be capable of maintaining a temperature of at least about 60-100°C, 65-95°C, 70-90°C, 75-85°C or about 80°C for a sustained period. In some embodiments, the heat source may be capable of heating the smokeable material so that at least a portion of the smokeable material maintains a temperature of at least about 60-100°C, 65-95°C, 70-90°C, 75-85°C or about 80°C for a sustained period. In some embodiments, that sustained period may be a period of about 1-10 minutes, 2-9 minutes, 3-8 minutes, or about 4-7 minutes.

In some examples, the exothermic reaction is a water-activated reaction, wherein water, or an aqueous solution or suspension, is added to one or more reagents in order to initiate an exothermic chemical reaction. Herein, the water or aqueous solution or suspension is referred to as the "activating reagent".

In other examples, the exothermic reaction may be a reaction between two or more reagents, none of which is water. For example, the exothermic reaction may include an organic liquid such as acetic acid as the activating reagent.

In some examples, the exothermic reaction used does not involve hazardous reagents or produce hazardous products, including any gas that may be given off during the exothermic reaction.

In some examples, the reagents which combine with water to cause a water-based exothermic chemical reaction include calcium oxide (CaO), sodium hydroxide (NaOH), calcium chloride (CaCl₂) and magnesium sulphate (MgSO₄). Other reagents for the exothermic reaction may also be used and represent embodiments of this invention. In some examples, these reagents are provided in solid form, for example in the form of a powder, granules, pellets or chunks, although they may also be used in other forms. Herein, these reagents are referred to as the "reactant".

The heat generation profile, that is, the amount of heat generated by the reaction of these reactants with water, the speed of heat generation and the duration of heat generation, will all be dependent upon a number of factors. For example, the heat generation profile may be adjusted by adjusting the ratio of water to reactant, the form of the reactant, in particular the particle size of the reactant and whether it is presented in powder, granular or pellet form, as well as the ratio and mixture of particle sizes. The speed and uniformity of the wetting of the reactant by the activating reagent will also influence the heat generation profile, as will the purity of reactants and the distribution of reactants. In addition, the nature of the reaction, i.e. the choice of reactant, will obviously affect the heat generation profile. One or more reactions may be utilised, either separately or together (provided that combining reactants/reactions is non-hazardous). Separate reactions may occur simultaneously or they may be staggered or sequential.

In some examples, the reactant is provided in particulate form, for example in the form of a powder, granules or pellets. The size of these particles may be selected to provide a desired heat generation profile. In particular, the use of smaller particles may provide faster initial heat generation whilst larger particles (in the range 1.6-3.0 mm) provide longer timescales for heat generation.

Rapid initial heat generation may not be coupled with sustained heat generation. Therefore, in some embodiments, the reactant does not consist essentially only of a powder, the powder having, for example, a mean particle size of less than 1 mm. Rather, in some examples, the reactant may comprise a combination of a powder (as defined above) and particles of greater particle size, such as pellets and/or granules. In some examples the ratio of powder to particles of greater particle size, such as pellets and/or granules may be from 3:1 to 1:3, or from 2: to 1:2, or from 1.5:1 to 1:1.5 by weight.

Where a combination of different forms of the reactant is used, it may be beneficial to provide the different forms in separate sections or layers, optionally in alternating layers, as this has been shown to aid temperature generation and promotes longevity of heat generation. In other examples, the different forms of reactant may be mixed and, optionally, uniformly mixed.

In some examples, the apparatus includes between about 0.5 and about 5 g of the reactant(s). In some embodiments, the mass of reactant is between about 1 and 4 g or between about 2 and 4 g or between about 2 and 3 g. In some embodiments, the apparatus includes between about 0.5 and 2 g of the reactant(s).

In some examples, to aid sustained heat generation the heat source consists of a combination of granules (1.6-3.omm in size) and fine powder in the ratio of 1.45g granules to ig fine powder. In some particular embodiments, the granules are placed towards the end of the apparatus with the mouthpiece, with the fine powder being positioned towards the other end. Other arrangements, including a homogeneous mixture of the different particle sizes, may also be used.

The ratio of activating agent to the reactants is also essential for the sustained heat generation and longevity, as it dictates the temperature and duration of the exothermic reaction. In some examples, the ratio of reactant to activating agent used may be from 3:1 to 1:3, or from 2:1 to 1:2, , or from 1.5:1 to 1:1.5, or from 1.25:1 to 1:1.25 or about 1:1 by weight.

In some examples, the reactant combined with water or an aqueous activating reagent may be CaO or NaOH, as these reactants can provide the target temperatures and provide heat over the desired sustained periods of time.

A further key factor which will affect the heat generation profile will be the mixing with and penetration into the reactants of the activating reagent. In some examples, the storage and/or release of the activating reagent is configured to ensure that the reagent quickly contacts as much of the reactant as possible.

In some examples, rapid mixing of the reactants and the activating reagent is achieved by introducing the activating reagent so that it contacts the reactant at a number of locations. This may be done, for example, by releasing the activating reagent through a plurality of openings or through a large (for example, elongate) opening, such as a slit. These openings or opening may be positioned to allow the activating agent to be quickly dispersed throughout the reactant. Alternatively or in addition, the activating reagent may be forcefully driven into the reactant under pressure, for example by injection.

The volume of activating reagent to be added is also a factor. Enough needs to be added to the reactant to achieve the target temperature as rapidly as possible and to ensure that all of the stored reactant undergoes the exothermic reaction. In addition, in some examples it may be desirable to keep the apparatus as small and/or light as possible, which will involve avoiding including an excessive amount of the activating reagent. Including more activating reagent than is necessary may also adversely affect heat generation, slowing the heat generation or reducing the temperature reached. In some examples, the apparatus includes between about 0.5 and 5 ml of water or an aqueous solution or suspension as the activating reagent. In some examples, the volume of activating reagent is between about 1 and 5 ml, about 1.5 and 4.5 ml, about 2 and 4 ml, or between about 2.5 and 3 ml. In some examples, the apparatus includes between about 0.5 and 2 ml of the activating reagent.

In other examples, the heat is produced by an air-based exothermic chemical reaction. For example, the reagents may be iron and oxygen. Upon exposure of the iron to oxygen in the air, exothermic oxidation of the iron occurs, generating heat. In some examples, the reaction mixture may further comprise water, activated carbon (to speed up reaction), vermiculite (which acts as a water reservoir) and salt (which acts as a catalyst). In some examples, the iron is provided in finely divided form, to increase the surface area available to react with the oxygen. The form of the iron, for example, its particle size, and the mixing of the iron with air upon actuation, will affect the heat generation profile, which may be optimised for the desired effect.

In embodiments of the invention, the heat provided by the heat source is produced by phase change reaction. This type of heat source may provide a lower temperature than the above described water- or air-based exothermic chemical reactions. As such, an apparatus having a heat source which involves a phase change is referred to herein as a low temperature device.

In some embodiments, the heat source of a low temperature device with a heat source involving a phase change is capable of heating at least a portion of the smokeable material to a temperature of about 40-80°C. In some embodiments, the heat source is heated to a temperature of about 40-80°C upon actuation. In further embodiments, the temperature of the heat source and/or of at least a portion the heated smokeable material is about 40-80°C, about 40-70°C, about 40-65°C or about 40-60°C. In some embodiments, the target temperature reached may be in the region of about 50-55°C.

Alternatively or in addition, the heat source may be capable of maintaining a temperature of at least or about 40-80°C, about 40-70°C, about 40-65°C or about 40-60°C for a sustained period. In some embodiments, that sustained period may be a period of 1-15 minutes, 2-10 minutes, or 3-8 minutes. In some embodiments, the sustained period is approximately 7 minutes. In other embodiments, the sustained period is about 3 to 4 minutes.

In some embodiments, the phase change is from liquid to solid. This may be particularly suited to the use in the apparatuses present invention. However, materials with other phase changes may be used.

There are several classes of phase change materials (PCMs). The two which are probably most suitable for use in the apparatuses of the present invention are paraffin waxes and hydrated salts, although paraffin waxes are challenging to supercool. Suitable phase change materials must meet two requirements: 1) melting temperature in the correct range; and 2) stability as a supercooled fluid at room temperature and/or at the possible storage temperatures.

Possible hydrated salts which may be used as heat generating phase change materials include sodium acetate trihydrate, sodium hydroxide monohydrate, barium hydroxide octahydrate, magnesium nitrate hexahydrate and magnesium chloride hexahydrate. Magnesium compounds are frequently unstable at room temperature, which can make them less attractive for use in the present invention, although gelation agents may be added to stabilise the phase change material. Sodium acetate trihydrate is a good candidate for use as a phase change material. It is stable at room temperature and is non-hazardous. In some embodiments, an additive may be added to the PCMs to improve performance. For example, a thickening agent may be added.

The maximum temperature generated by phase change materials is dependent upon their phase change temperature, for example, their melting temperature where the phase change is from liquid to solid. In addition, the heat generation profile, including the period over which heat is generated and a temperature within a particular range may be maintained, may be controlled by controlling the rate at which heat is transferred away from the phase change material. This will be discussed in greater detail below.

Where the phase change material comprises sodium acetate trihydrate, the formulation may affect the temperature generated. For example, the higher the water content of the formulation, the lower the temperature that may be achieved by the phase change of the sodium acetate trihydrate. This may be the case with other phase change materials. In addition, the formulation may improve stability of the phase change material in the liquid state, and may also increase the shelf life of the phase change heat source, whether already associated with the apparatus or as a separate product to be associated with the apparatus prior to use.

In some examples, the apparatus may include a combination of two or more different types of heat sources. For example, the apparatus may include both a heat source which is based upon an exothermic chemical reaction and a heat source which is based upon an exothermic phase change. Alternatively or in addition, the apparatus may include two or more different exothermic chemical reactions. This may be a combination of two or more different water-based exothermic reactions or a combination of water-based, non-water based and/or air-based chemical reactions. Combining different heat sources may be a means of achieving a desired heat generation profile with the desired rapid onset, target temperature and duration of heat generation.

An apparatus embodying the present invention comprises a housing within which the heat source is held in a heat source chamber, and a smokeable material is held in a heating chamber, the heat source chamber and the heating chamber being arranged to allow transfer of heat from the heat source chamber to the smokeable material, so that at least one component of the smokeable material may be volatilised. In addition, the apparatus includes a mouthpiece through which the volatilised component(s) may be inhaled. The apparatus also includes an actuating mechanism which will allow a user to activate the heat source.

In some embodiments, the heat source and/or smokeable material may be provided separately and may be introduced into the apparatus before use. This allows the apparatus to be reused, the heat source and/or smokeable material being replaceable and/or disposable and/or reusable elements. The heat source and/or the smokeable material may be provided as a cartridge which may be loaded into the apparatus. The cartridge may comprise one or both of the heat source and the smokeable material. In some embodiments, it may be possible to recharge and reuse the phase change material heat source. This may be done by heating the material by any suitable means, for example by an electrical heating step. The recharging may be carried out whilst the used heat source is still associated with the apparatus or it may involve removing the heat source from the apparatus. In other embodiments, the apparatus is for a single use and the whole apparatus is disposed of following use.

In some embodiments, the heat source is arranged to rapidly transfer heat to the smokeable material. For example, the heat source may be at least partially surrounded by the smokeable material. This arrangement may promote a rapid and uniform transfer of the heat produced by the heat source to the smokeable material. Alternatively or in addition, the smokeable material may be at least partially surrounded by the heat source.

In some examples, a high temperature device has a heat source which is partially or completely surrounded by the smokeable material which is to be heated. Alternatively or in addition, the smokeable material may be at least partially surrounded by the heat source.

In some embodiments, a low temperature device having a heat source which is an exothermic phase change has the heat source configured to partially or completely surround the smokeable material to be heated. This arrangement may be combined with an insulating layer surrounding the outer surface of the heat source, to help direct the heat generated to the smokeable material. Alternatively or in addition, the heat source may be at least partially surrounded by the smokeable material.

In embodiments of the invention, the device is configured so that the phase change material is at least partially visible from outside the device. This allows the user to determine whether or not the device has been previously been used (i.e. whether the phase change material has previously been activated and has already solidified). Additionally, the visible phase change material means that the user has a visual indication whether or not the actuation of the device has successfully triggered the phase change. The appearance of the phase change material can also act as a visual indicator to the user that the heat source is heating the smokeable material so as to volatilise components and deliver them in an inhalable form, i.e. it may act as a visual indicator that the device is ready for use.

In some embodiments, the phase change material is held in a heat source chamber within the device. In some embodiments, at least a portion of the wall of the heat source chamber is clear or translucent, so that the phase change material may be viewed. Where the device includes a housing, in some embodiments, at least a portion of the housing is clear or translucent, so that the phase change material may be viewed. In some embodiments, at least a portion of the housing and a portion of the walls of the internal chambers of the device, including the heat source chamber and the heating chamber (which may hold or receive the smokeable material), are clear or translucent, so that the phase change material and the smokeable material may be viewed from outside the device.

The actuating mechanism may comprise a button, optionally a spring-loaded and/or sliding button. Various other actuating mechanisms are also envisaged. For example, the actuating mechanism may be configured to be twisted or squeezed to activate the apparatus and/or the heat source. In one embodiment, the actuating mechanism may require the user to twist, tap, press or squeeze a portion of the device, such as one end, optionally not the end with the mouthpiece. In other embodiments, a part of the device may be removed in order to activate the heat source.

In some embodiments, actuation may result in the mixing of the reactants and the activating agent. In other embodiments, actuation may result in the provision of a nucleation point to trigger crystallisation of a phase change material. This may, for example, involve dropping powdered reactant into a super-cooled liquid reactant. In yet further embodiments, a shearing motion may initiate the phase change.

In some embodiments, activation of the phase change material may be by a seed crystal which is brought into contact with the phase change material to trigger the phase change. In some embodiments, the seed crystal may be stored away from the phase change material but is moved to a position in contact with the phase change material by the actuating mechanism. Alternatively, the phase change could be triggered by a sharp point or clicker providing the nucleation.

In some embodiments, the apparatus has a cylindrical shape and may have similar dimensions to conventional smokeable products such as cigarettes, cigars and cigarillos. In alternative embodiments, the apparatus may resemble a pipe. In other embodiments the apparatus may be of any desired shape.

In some embodiments, the apparatus comprises about 100-800 mg of smokeable material, or 100-400 mg of smokeable material. In some embodiments, 100-200 mg smokeable material is included, whilst in other embodiments 200-300 mg smokeable material is included. Alternatively or in addition, the smokeable material may be included in an amount suitable to provide about 0.01-1 mg nicotine in vapour form, or 0.01- 0.5 mg. In some embodiments, the amount of nicotine emitted from the apparatus may be about 0.1-0.3 mg, for example from 600 mg of tobacco. The amount of nicotine produced in vapour form may be adjusted by changing the amount of smokeable material, the nature of the smokeable material and/or the temperature to which the smokeable material is heated and for how long. The nicotine delivery is measured under ISO conditions, however it should be understood that the apparatus may emit different amounts of nicotine dependant on how the consumer uses the product.

In some examples of the high temperature device where the smokeable material is heated to a temperature in the range of about 60-100°C, the smokeable material included in an amount of between about 250-300 mg may yield about 0.1-0.3 mg nicotine in vapour form. In some embodiments of the low temperature device where the smokeable material is heated to a temperature in the range of about 40-80°C, the smokeable material included in an amount of between about 250-300 mg may yield about 0.03-0.1 mg nicotine in vapour form.

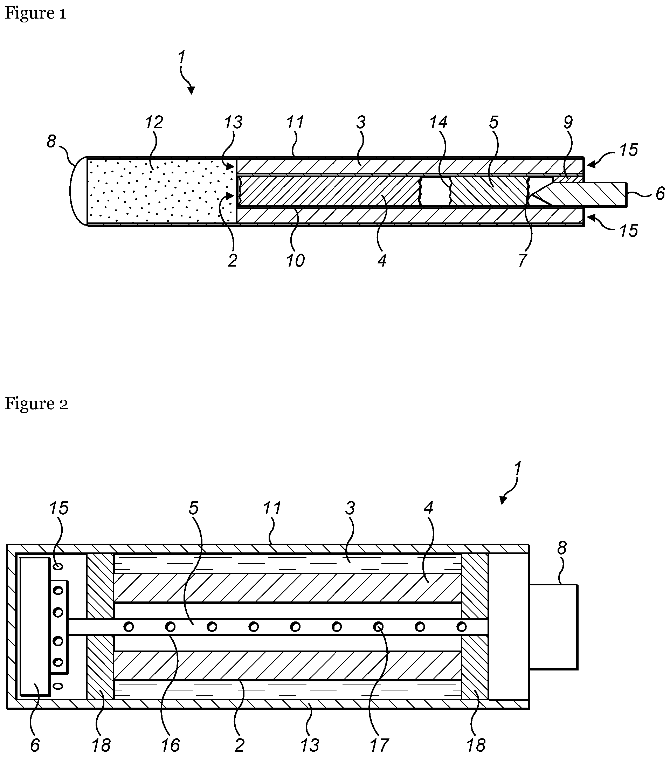

Figure 1 shows an apparatus 1 for heating smokeable material 3 wherein the heat is provided by an exothermic water-based chemical reaction, a so-called high temperature device.

The apparatus 1 comprises a heat source chamber 2, separately holding two heat source reagents (a reactant 4 and an activating reagent 5). The heating chamber 13 is configured to receive smokeable material 3 so that the smokeable material 3 can be heated in the heating chamber 13. For example, the heating chamber 13 may be located adjacent to the heat source chamber 2 so that thermal energy from the heat source heats the smokeable material 3 therein to volatilise aromatic compounds and nicotine in the smokeable material 3 without burning the smokeable material 3. All of the components may be held within a housing 11. A mouthpiece 8 is provided through which a user of the apparatus 1 can inhale the volatilised compounds during use of the apparatus 1. The apparatus 1 includes a flowpath for air which enters the heating chamber 13 via inlets 15. In the heating chamber 13, the air picks up the volatilised compounds generated by heating the smokeable material 3 before reaching the mouthpiece 8 from which the air and entrained volatilise compounds may be inhaled. The flowpath is isolated from the heat source chamber 2.

The smokeable material 3 may comprise a tobacco blend.

A housing 11 may contain components of the apparatus 1 such as the heat source reagents 4,5 located in the heat source chamber 2 and the smokeable material 3 in the heating chamber 13. As shown in Figure 1, the housing 11 may comprise an approximately cylindrical tube with the heat source chamber 2 aligned along the central longitudinal axis of the housing 11 and surrounded by the heating chamber 13 holding the smokeable material 3. Figure 1 also shows an optional filter 12 positioned at the mouth-end of the apparatus (which is the end of the apparatus which includes the mouthpiece).

In order to ensure that the exothermic chemical reaction only starts when the actuating mechanism is actuated, at least one of the reagents is stored separately from the other reagent(s). Actuation of the actuating mechanism causes the reagents to be combined, activating the exothermic reaction.

In one possible arrangement, as illustrated in Figure 1, the reagents 4,5 are stored in a heat source chamber 2 and are separated by a rupturable element 14 which is ruptured upon actuation of the actuating mechanism 6 to allow the reagents 4,5 to combine. This rupturable element 14 may comprise, for example, a wall, a portion of a wall, or some other structure which separates the reagents and which is susceptible to failure or may be removed, for example, upon application of force in a certain manner, or which may be pierced, punctured, dissolved or otherwise partially or completely removed to allow mixing of the reagents. In Figure 1, the rupturable element is a flexible bag 14 and the illustrated actuating mechanism comprises a piercing element 7 to pierce or rupture the flexible bag 14 in such a way that the activating reagent 5 mixes with the reactant 4.

The heat source chamber 2 may extend along a longitudinal axis of the apparatus 1. The heating chamber 13 may also extend along a longitudinal axis of the apparatus 1 and may be located adjacent to the heat source chamber 2. For example, the heat source chamber 2 shown in Figure 1 extends substantially along the central longitudinal axis of the apparatus 1 and the heating chamber 13 is located around its longitudinal surface. If the heat source chamber 2 is substantially cylindrical, as shown in Figure 1, then the longitudinal surface around which the heating chamber 13 extends is a circumferential surface of the heat source chamber 2. In this type of configuration, the heating chamber 13 may comprise a co-axial layer around the heat source chamber 2. This provides an annular space around the heat source chamber 2 into which the smokeable material 3 can be inserted for heating.

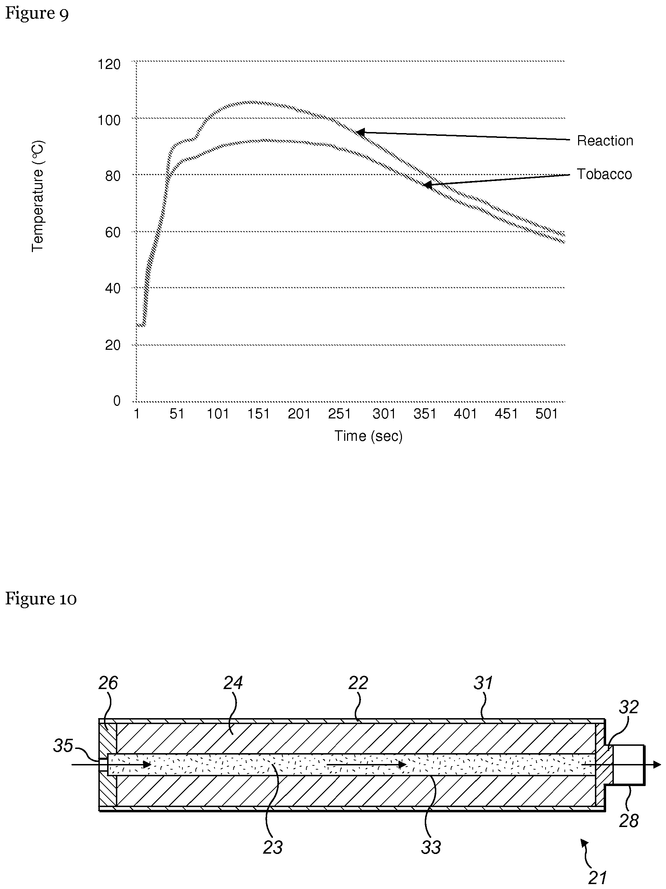

An alternative arrangement is for the positions of the heating chamber 13 and the heat source chamber 2 to be reversed, so that the heating chamber 13 is located along the central longitudinal axis of the apparatus 1 and the heat source chamber 2 is located annularly around it as a co-axial layer. This general arrangement with the heat source surrounding the smokeable material is illustrated in Figure 10 which is a low temperature device and is discussed below.

Figure 2 shows an alternative apparatus 1 for heating smokeable material 3 using an exothermic water-based chemical reaction, an alternative high temperature device. The apparatus 1 comprises a heat source chamber 2, holding a heat source reactant 4 and a separate reagent storage chamber 16 holding the activating reagent 5, such as water. The heating chamber 13 is configured to receive smokeable material 3 so that the smokeable material 3 can be heated in the heating chamber 13. All of the components are held within a housing 11. A mouthpiece 8 is provided through which a user of the apparatus 1 can inhale the volatilised compounds during use of the apparatus 1. At the other end of the illustrated apparatus 1 is an actuating mechanism 6 in the form of a button which may be depressed in order to release the activating reagent from the reagent storage chamber 16.

The reagent storage chamber 16 is a tube provided with a series of sealed orifices 17 along its length. The orifices 17 are opened upon actuation of the actuating mechanism 6 to allow the activating reagent 5 to leave the tube and enter the heat source chamber 2 and mix with the reactant 4 to start the exothermic reaction. Where the activating reagent is forced out of the tube under pressure, for example as a result of the action of the actuating mechanism, the activating reagent may be forced out of the one or more orifices, which may facilitate rapid and uniform mixing with the reactants in the heat source chamber. This in turn may result in the target temperature being reached sooner after actuation of the actuating mechanism.

In other examples, the apparatus comprises a tube with one or more orifices, but the activating reagent is not stored within this tube. Rather, the activating reagent is stored in a separate reagent storage chamber. Upon actuation of the apparatus, the activating reagent is moved from the reagent storage chamber and passes into the tube and through the one or more orifices into the heat source chamber where it mixes with the reactant and the exothermic reaction starts. Where the activating reagent is fed into the tube under pressure, it may be forced out of the one or more orifices, which may facilitate rapid and uniform mixing with the reactants in the heat source chamber. This in turn may result in the target temperature being reached sooner after actuation of the actuating mechanism.

In some examples, the length of the housing 11 may be approximately 130mm. The length of heat source chamber 2 may be approximately the same as the length of the heating chamber 13. The heat source chamber 2 and heating chamber 13 may run substantially the entire length of the apparatus, thus having a length of approximately 130 mm in some embodiments. In other examples, as shown in Figure 1, the apparatus may include a filter section 12 at the mouth end of the apparatus, positioned along the central longitudinal axis of the apparatus and abutting one end of the heat source chamber 2 and the heating chamber 13. Additionally or alternatively, one or more filters may be provided in other positions, as discussed in greater detail below.

In some examples, the diameter of the housing 11 may be, for example, between approximately 7 mm and approximately 18 mm. In some examples, the diameter of the housing 11 may be between approximately 15mm and approximately 18 mm. In some examples, the diameter may be the same at each end of the housing, whilst in other examples, the diameters may be different. For example, in some examples, the diameter of the housing at the mouth end 8 may be 15 mm whilst the diameter of the other end of the housing may be 15 mm.

The diameter of the heat source chamber 2 may be between approximately 1 mm and approximately 6 mm. In some embodiments, it may be possible for the diameter of the heat source chamber to be as small as about 1 mm to about 1.2 mm. The rest of the diameter of the housing may be occupied by the heating chamber 13, which is arranged circumferentially around the heat source chamber 2.

The housing 11 is suitable for being gripped by a user during use of the apparatus 1 so that the user can inhale volatilised smokeable material compounds from the mouthpiece of the apparatus. The housing may be manufactured from or may comprise insulating material. This will reduce the surface temperature of the apparatus by preventing the transfer of heat. It is desirable that the surface of the apparatus is a temperature that is comfortable and safe to touch during use. Suitable insulation may include, for example, polystyrene, high density polyethylene (HDPE), polyvinyl chloride (PVC), poly(methyl methacrylate) (PMMA) which is also known as Perspex®, or acrylonitrile butadiene styrene (ABS). In some embodiments, a sleeve may be formed from any one or a combination of these or other suitable insulating materials to prevent the transfer of heat. In some embodiments, a sleeve made from a styrene foam may be used. In some embodiments, the main outer casing for the device can be constructed from aluminium or plastic materials.

The heat source chamber 2 containing the reagents 4,5 may be a substantially cylindrical, elongate chamber and the heating chamber 13 is located around a circumferential, longitudinal surface of the heat source chamber 2. The heating chamber 13 and smokeable material 3 therefore comprise co-axial layers around the heat source chamber 2. However, as will be evident from the discussion below, other shapes and configurations of the heat source chamber 2 and heating chamber 13 can alternatively be used, for example to increase the surface area of the interface between these, for example in order to enhance heat transfer.

For example, in some embodiments, the heat source chamber 2 comprises a spirally shaped chamber. The spirally shaped chamber is surrounded by the heating chamber 13 comprising the smokeable material 3 to be heated. The spiral configuration of the heat source chamber 2 provides an increased area of interface between the heat source and the smokeable material 3, enhancing transfer of heat to the smokeable material 3.

The heat generated by the heat source will heat the adjacent heating chamber and the smokeable material held therein. In some embodiments, the heat source chamber 2 and the heating chamber 13 are in close proximity, to assist the transfer of heat. In some embodiments, the wall 10 between the heat source and the smokeable material is thermally conductive, to assist effective thermal transfer from the heat source to the smokeable material 3 inside the heating chamber 13, thereby causing the smokeable material 3 to be heated to a temperature which is sufficient to volatize components of the smokeable material 3 for inhalation through the mouthpiece 8.

In the example illustrated in Figures 1 and 2, the apparatus provides volatilised components of smokeable material 3 for inhalation. The heating chamber 13 containing the smokeable material 3 is fluidly connected with the mouthpiece 8. The air flowpath is, however, in at least some embodiments isolated from the heat source chamber 2. This is particularly desirable where the heat source generates a gas which should not be inhaled by the consumer.

As illustrated in Figure 1, the apparatus may comprise a filter 12, including a filter material such as cellulose acetate tow, which may be provided in the form of a wrapped plug. The filter 12 may be positioned between the heating chamber 13 and the mouthpiece 8. The filter 12 may provide physical and/or chemical filtration. In some embodiments, it is provided to prevent particles of the smokeable material from becoming entrained in the air flow and being inhaled. Other suitable filters may comprise a wire gauze or the like. Alternatively or in addition, a filter may be included to filter the air being drawn into the apparatus, in which case, the filter optionally may be positioned at the air inlet to filter the air before it enters the heating chamber 13.

The inclusion of a filter comprising a plug of filter material such as cellulose acetate tow may be useful in controlling the pressure drop of the apparatus. If provided for that purpose, the filter may, in some embodiments, be positioned at any point along the air flow path through the apparatus and could be positioned at or near the air inlet so that it affects the air flow before it enters the heating chamber and entrains the volatilised components.

A user of the apparatus 1 can inhale the volatilised components from the mouthpiece 8 when the smokeable material 3 is heated inside the heating chamber 13 to a temperature which is sufficient to volatilise the smokeable material components. In some embodiments, the air drawn through the apparatus by the user passes through a filter such as a cellulose acetate tow filter. Such a filter may be used to control the pressure drop and/or to filter the air, for example, preventing the inhalation of particles of smokeable material.

In some examples, the heat source chamber 2 may include a void prior to actuation in order to allow for the expansion of the heat source material upon reacting.

Additionally or alternatively, as shown in Figure 1, the apparatus 1 may include a vent to allow the escape of gases formed by the exothermic reaction. In some embodiments, the apparatus may include a vent filter 9 at an exhaust vent from the heat source chamber 2. The vent may be positioned away from the mouthpiece to prevent inhalation of any vented gases by the user. The vent filter 9 may trap certain components of the exhaust gases, such as steam, whilst allowing any pressure built up within the heat source chamber as a result of the exothermic reaction to be released. Suitable filter materials for this may, for example, include materials providing a tortuous path, steel wool, filter tow (such as cellulose acetate tow), aluminium foil, high density polyethylene (HDPE), metal gauze, or moulded plastic gauze.

It is desirable to heat the smokeable material 3 to within a desired volatilising temperature range and to maintain the temperature of the smokeable material 3 in the desired volatilising temperature range for a predetermined sustained period. An example of a temperature range in which components of smokeable material such as tobacco are volatized is between approximately 40°C and approximately 250°C, although other ranges may also be suitable. The sustained period may be approximately the same length as the period taken to smoke an average cigarette. For example, the sustained period may be between approximately 4 and 8 minutes, such as approximately 5 to 7 minutes.

As previously described, heat from the heat source chamber 2 is transferred into the smokeable material 3 in the heating chamber 13, causing at least one component of the smokeable material 3 to be volatilised when the smokeable material 3 reaches a volatilising temperature. Optionally, the apparatus 1 may comprise a temperature sensor (not shown), for example comprising a thermocouple, which is configured to detect when the heating chamber 13 and/or smokeable material 3 in the heating chamber 13 has reached a predetermined volatilising temperature. The temperature sensor may comprise or communicate with an alarm, indicator light or some other suitable alerting mechanism, which is configured to inform a user that the volatilising temperature has been reached and thus volatilised components of the smokeable material 3 are available for inhalation. Such an alert may, for example, utilise thermochromic ink which indicates when the target temperature has been reached. In some embodiments, the alert may also indicate when the apparatus has dropped below the optimum operating temperature. Again, a thermochromic ink may provide such an indication.

In some embodiments, a locking mechanism may be included which prevents use of the apparatus before the volatilising temperature has been reached and the volatilised components of the smokeable material 3 are available for inhalation. Such a locking mechanism may, for example, include a bimetallic strip connected to a valve, whereby the strip has a configuration which locks the valve until it reaches a target temperature and changes shape so as to allow the valve to open. The locking mechanism may be configured to return to the locked configuration once the apparatus has dropped below the optimum operating temperature following use.

Additionally or alternatively, the apparatus 1 may comprise a timer (not shown) which is configured to measure the length of time from actuation of the actuating mechanism and to cause the user to be alerted when a predetermined heating time, which is known to correspond to the volatilising temperature being reached in the heating chamber 13, has elapsed. The timer could additionally or alternatively indicate when the apparatus has stopped producing vapour for the consumer to inhale, again based upon a predetermined time period having elapsed since actuation of the apparatus.

Components of the smokeable material 3 which have been volatilised, or are subsequently volatilised, by the heat released from the heat source chamber 2 can be inhaled from the mouthpiece. This allows a user of the apparatus 1 to inhale volatilised components from the mouthpiece 8 in a manner which is similar to how a user would inhale smokeable material components from a smoking article such as a cigarette.

In some embodiments, the apparatus 1 may comprise air inlets 15 which allow external air to be drawn into the heating chamber 13 within the housing 11 and through the heated smokeable material 3 during puffing. The air inlets 15 may comprise apertures in the housing 11 and may be located upstream from the smokeable material 3 and heating chamber 13 towards the opposite end of the housing 11 to that with the mouthpiece. This is shown in Figures 1 and 2. Air drawn in through the inlets 15 travels through the heated smokeable material 3 and therein is enriched with smokeable material vapours, such as aroma vapours, before being inhaled by the user at the mouthpiece 8. Optionally, as shown in Figure 1, the apparatus 1 includes a filter 12 through which the air enriched with smokeable material vapours travels before exiting the apparatus 1 at the mouthpiece 8. In other embodiments, the air enriched with smokeable material vapours does not pass through a filter before exiting the apparatus 1 through the mouthpiece 8. In some embodiments, the air flows through a filter before entering the heating chamber 13.

The optionally insulated heating chamber 13 may comprise inlet and outlet valves which hermetically seal the heating chamber 13 when closed. The valves can thereby prevent air from undesirably entering and exiting the chamber 13 and can prevent vaporised components of the smokeable material from exiting the chamber 13 until required or desired. The inlet and outlet values may, for example, be provided in the insulation.

In some embodiments, the valves may be closed by a controller so that all volatilised substances remain contained inside the chamber 13 between puffs. The partial pressure of the volatized substances between puffs reaches the saturated vapour pressure and the amount of evaporated substances therefore depends only on the temperature in the heating chamber 13. This helps to ensure that the delivery of volatilised nicotine and aromatic compounds remains constant from puff to puff. During puffing, the controller is configured to open the valves so that air can flow through the heating chamber 13 to carry volatilised smokeable material components to the mouthpiece 8. A membrane can be located in the valves to ensure that no oxygen enters the heating chamber 13. The valves may be breath-actuated so that the valves open in response to detection of a puff at the mouthpiece 8. Alternatively, the valves may be caused to open automatically by the suction force generated by a user when drawing on the mouthpiece 8. The valves may close in response to a detection that a puff has ended. The apparatus may be provided with a puff sensor to trigger opening and closing of the valves at appropriate times. Alternatively, the valves may close following the elapse of a predetermined period after their opening. The predetermined period may be timed by the controller. Optionally, a mechanical or other suitable opening/closing means may be present so that the valves open and close automatically. For example, the gaseous movement caused by a user puffing on the mouthpiece 8 may be used to open and close the valves. Therefore, the use of the controller is not necessarily required to actuate the valves. Where the heating chamber 13 is sealed so that volatilised components may not be immediately released, it may be desirable to include means for ensuring that the volatilised components do not condense in the heating chamber 13. This may involve further heating to keep the vapour above the temperature at which it condenses.

Thermal insulation may be provided between the smokeable material 3 and the external surface of the housing 11 to reduce heat loss from the apparatus 1 and therefore improve the efficiency with which the smokeable material 3 is heated. For example, referring to the apparatus illustrated in Figure 1, a wall of the housing 11 may comprise a layer of insulation (not shown) which extends around the outside of the heating chamber 13. The insulation layer may comprise a substantially tubular length of insulation located co-axially around the heating chamber 13 and smokeable material 3. It will be appreciated that the insulation could also be comprised as part of the heating chamber, which may be provided as a removable cartridge, in which the insulation would be located co-axially around the outside of the smokeable material 3.

In some embodiments the thermal insulation may, for example, comprise vacuum insulation which is configured to insulate the apparatus 1 by providing an evacuated region between the heating chamber 13 and the external surface of the housing 11 to thereby substantially prevent heat losses by conduction and convection. The housing 11 and/or heating chamber 13 may additionally or alternatively be provided with an infrared reflective layer located between the smokeable material 3 and the external surface of the housing 11 so as to prevent losses by thermal radiation. The reflective layer may optionally be provided on or in the thermal insulation layer.

The thermal insulation also ensures that the external surface of the housing 11 is substantially not heated by the heat source 4, 5 and therefore that the external surface of the housing 11 remains at a temperature which is comfortable for a user to grip the housing 11 during inhalation of volatilised components from the mouthpiece 8. Alternatively of additionally, the housing may be surrounded by a thermally insulating sleeve. This sleeve may reduce the temperature at the surface of the apparatus and/or may provide the apparatus with a desired look or feel.

In the example illustrated in Figure 1, the heat source reagents comprise a reactant 4, such as calcium oxide (CaO) in particulate solid form (e.g. in the form of powder and/or chunks), and a second (activating) reagent which is water 5. In the illustrated example, the water 5 is held in a sachet or flexible bag 14. In alternative examples, the reactant 4 may be provided in the form of a solid cylinder, or may be an open annular structure.

The exothermic reaction may be triggered by the use of the actuating mechanism. In the illustrated example, the actuating mechanism comprises a piercing member 6 which is moveable from a resting position, as shown, to an actuating position in which it has been moved by sliding into the apparatus 1 so that the piercing end 7 contacts and punctures the flexible bag or sachet 14 holding the water 5. This causes release of the water into the heat source chamber where it will contact the other reagent 4, triggering the exothermic reaction and generating heat.

In alternative examples, the reagents 4, 5 are stored in the heat source chamber and are separated by a rupturable element which is ruptured upon actuation of the actuating mechanism to allow the reagents to combine. This rupturable element may comprise, for example, a wall, a portion of a wall, or some other structure which separates the reagents and which is susceptible to failure or may be removed, for example, upon application of force in a certain manner, or which may be pierced, punctured, dissolved or otherwise partially or completely removed to allow mixing of the reagents. In such examples, the actuating mechanism may be arranged to rupture the rupturable element. This rupturable element, located between the reagents in the heat source chamber, may be positioned towards the middle of the length of the apparatus, rather than at one end. Thus, the actuating mechanism may comprise a piercing mechanism which is actuated by a button, slider or other actuator, which is positioned on the outer surface of the housing of the apparatus.

In an alternative arrangement, at least one reagent is stored in the heat source chamber and at least one reagent is stored in a separate reagent storage chamber. The actuation of the actuating mechanism may then cause the transfer of the at least one reagent in the reagent storage chamber into the heat source chamber where resultant combination of reagents will cause an exothermic reaction.

One example of such an arrangement is illustrated in Figure 2. The reagent storage chamber 16 containing the activating reagent 5 is a tube extending longitudinally through the heat source chamber 2 containing the reactant 4. The tube is provided with sealed orifices along its length within the heat source chamber 2. Upon actuation of the actuating mechanism 6, the orifices 17 are opened to allow the activating reagent 5 to exit the reagent storage chamber 16 and to mix with the reactant 4 in the heat source chamber 2. The actuating mechanism may also apply pressure to force the actuating reagent 4 out of the chamber 16, thereby encouraging mixing with the reactant 4. In an alternative embodiment, the orifices 17 could be replaced with a slit which is sealed prior to actuation. In some embodiments, the reagent storage chamber 16 may be held in place by supports 18 positioned within the apparatus 1, for example at each end of the heat source chamber 2.

In an alternative arrangement, rather than the activating reagent 5 being held within a reagent storage chamber within the heat source chamber 2, the reagent storage chamber is outside the heat source chamber 2. The actuation of the actuating mechanism 6 may, for example, cause release of the activating reagent from the reagent storage chamber and its transfer to the heat source chamber. This transfer may be via a tube. For example, the activating reagent 5 may be held within the reagent storage chamber which is associated with or is within the actuating mechanism, so that the activating reagent is introduced into the heat source chamber 2 when the actuating mechanism is actuated. The actuation of the actuating mechanism may, for example, cause release of the activating reagent from the reagent storage chamber and its transfer to the heat source chamber 2, for example via a tube or other conduit. In some examples, the actuating mechanism may act like a syringe or the like, with a plunger mechanism pushing or injecting the activating reagent 5 through one or more orifices into the heat source chamber 2 where it will come into contact with the reactant 4, thereby triggering the exothermic reaction. The orifices may be positioned to enhance mixing of the activating reagent 5 and the reactant 4. In alternative examples, the actuating mechanism may act like a pipette, with the activating reagent 5 being pushed into the heat source chamber 2 as the reagent storage chamber is compressed or squeezed.

In some examples, the exothermic reaction may be a water-activated reaction, wherein water, or an aqueous solution or suspension, is the activating reagent which is added to one or more reactants in order to initiate an exothermic chemical reaction.

The suitability of various possible exothermic reactions involving water was assessed. A set of experiments was devised to evaluate the variables which affect the temperatures achieved by exothermic reactions, including the following variables: the mass of the reactant; the volume of water; the form of the reagent added; and the nature of the reaction vessel.

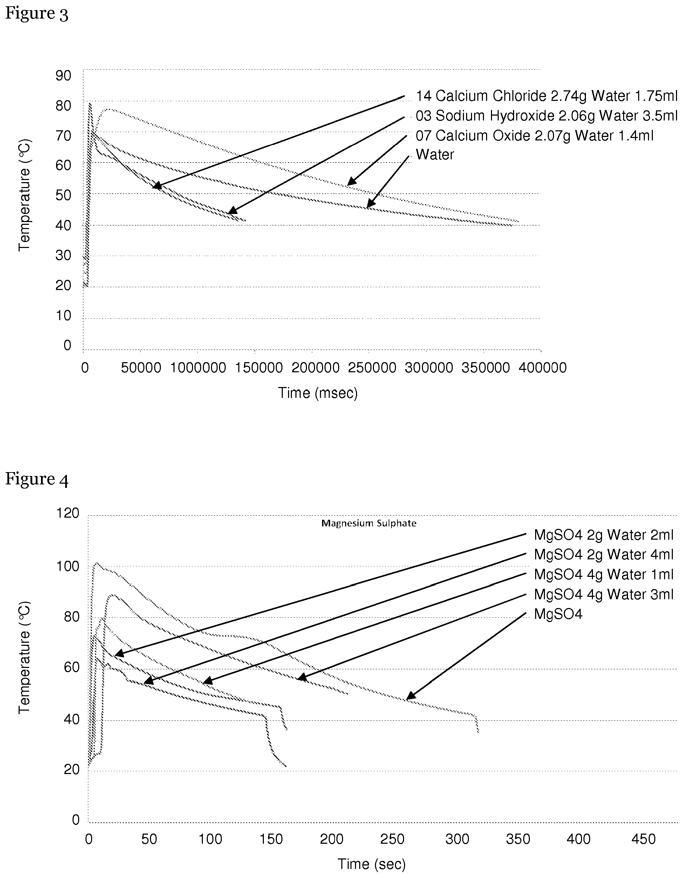

Figure 3 shows the results of the initial experiments in which CaCl₂, NaOH and CaO were tested as reactants. The reactions were carried out in a small glass beaker with no insulation. A thermocouple was used to log the temperatures. Based on the reaction stoichiometry, the minimum amount of reagents was added to make up to a maximum volume of approximately 7 ml for all of the reactions. The graph in Figure 3 charts the heat generation of three reactions: (i) 2.74 g CaCl₂ and 1.75 ml water; (ii) 2.06 g NaOH and 3.5 ml water; and (iii) 2.07 g CaO and 1.4 ml water. The initial experiments indicated that CaO and NaOH were closest to the proposed target temperature of 80°C.

Next, magnesium sulphate was tested, using varying ratios with water. As the results set out in the graph of Figure 4 show, the target temperature of 80°C was rapidly achieved with MgSO₄ as the reaction is initiated, but the temperature decay was rapid, which is not compatible with the desire to maintain the target temperature at 80°C for a period of at least 3 minutes, for example, for at least between 3 to 4 minutes, and possibly even longer for some examples.

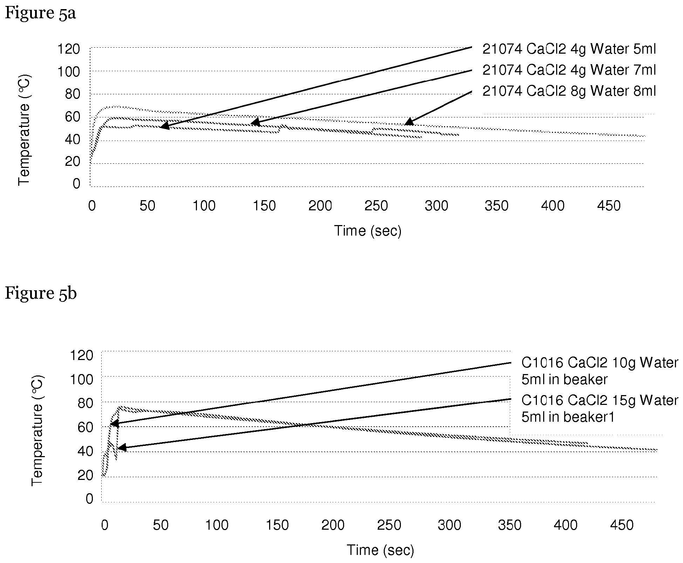

Further experiments were carried out to evaluate how the form of the reactant affects the heat generation profile. Two different forms of CaCl₂ were used. Firstly, granular CaCl₂ was tested (available from Sigma Aldrich under catalogue no. 21074), which had a particle size of 1-6 mm and included up to 3% water. Secondly, anhydrous CaCl₂ granular spheres were tested (available from Sigma Aldrich under catalogue no. C1016) which had a particle size of no more than 7 mm. The results are shown in Figures 5a and 5b, respectively. They demonstrate that the anhydrous form generated higher initial temperatures.

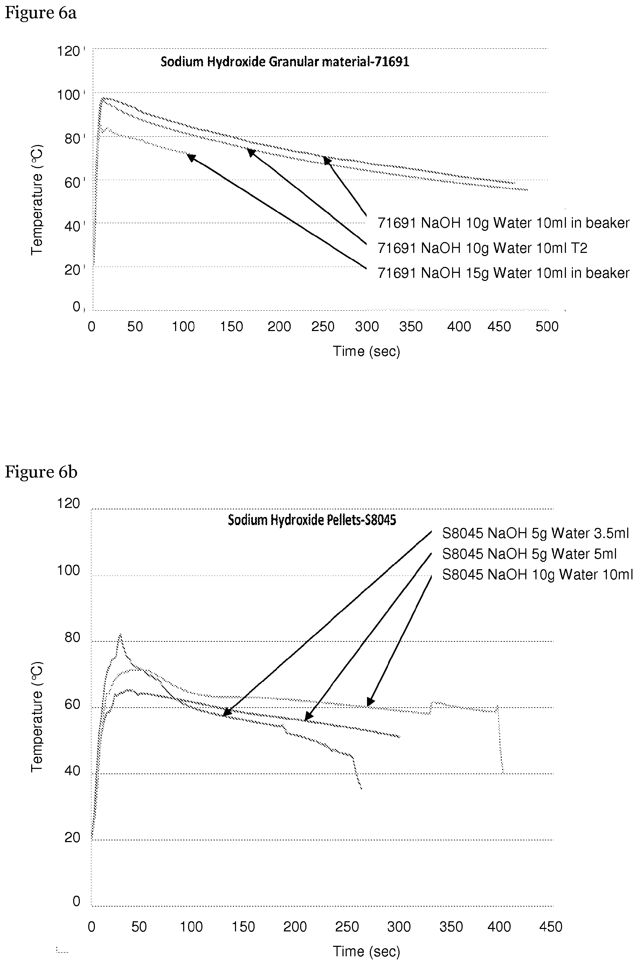

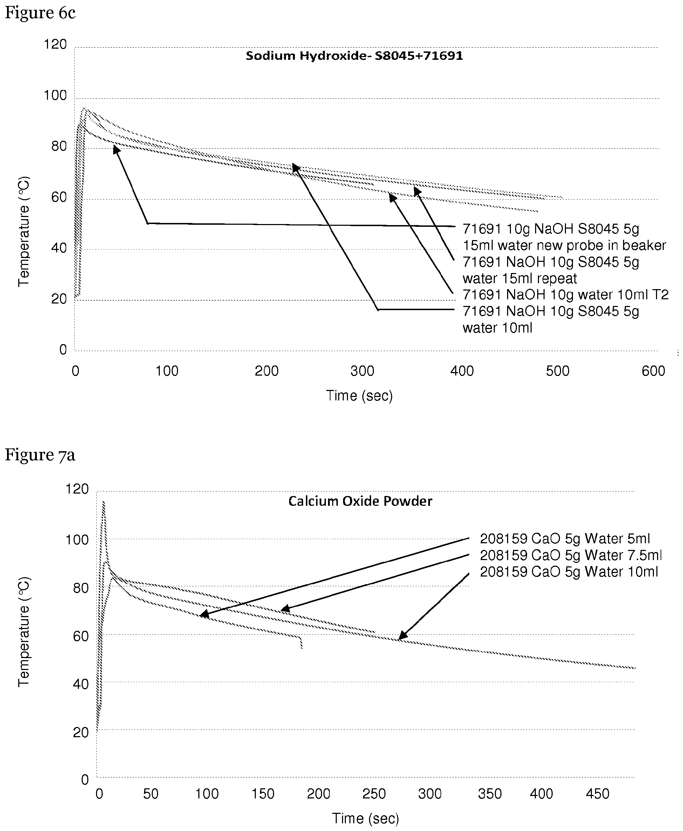

Further experiments were carried out to evaluate how the form of the reactant affects the heat generation profile. Two different forms of NaOH were used. Firstly, NaOH in the form of flakes was tested (available from Sigma Aldrich under catalogue no. 71691). Secondly, anhydrous NaOH pellets were tested (available from Sigma Aldrich under catalogue no. S8045). In addition, the experiment was carried out with a combination of these forms of NaOH. The results are shown in Figures 6a, 6b and 6c, respectively. The results indicate that the flakes of NaOH exhibited the highest temperatures and modulation. Combining the two forms of NaOH resulted in raised reaction temperature and slower temperature decay, i.e. a longer duration of heat generation.

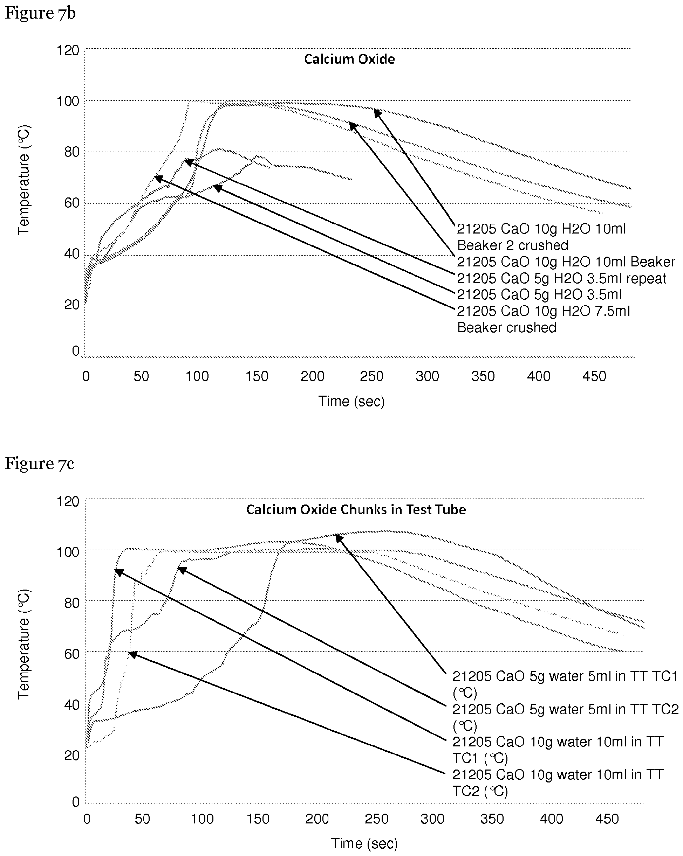

Further experiments were carried out to evaluate how the form of CaO affects the heat generation profile. Two different forms of CaO were used. Firstly, CaO in the form of powder was tested. Secondly, CaO chunks were tested. In addition, a further experiment was carried using the CaO chunks, but carrying out the reaction in a test tube, rather than a small beaker. This was to evaluate the effect on the heat generation profile of a change in the volume (and form) of the reaction vessel. The results are shown in Figures 7a, 7b and 7c, respectively. The results indicate that the CaO powder rapidly achieved a high temperature, but that this was not sustained and the temperature rapidly dropped. In contrast the reaction using CaO chunks was much slower to achieve the temperature peak, but the heat generation was more sustained. The effect of performing the reaction with the CaO chunks in a smaller reaction vessel was to speed up the initial heat generation, and the temperature was maintained well over the extended period of time.

These graphs demonstrate that the heat generation profile may be modified by using and/or combining different forms of the reactant, varying amounts of the reagents and the reaction vessel. This allows the temperature and duration of heat generation to be tailored, allowing the heat profile to be designed and controlled with some accuracy.

In some examples, the exothermic reaction reagents comprise CaO or NaOH and water. In some embodiments, the reagents comprise CaO and water. This reaction is particularly suited to delivering the desired high temperature over a sustained period of time. The use of CaO in a combination of particulate forms (i.e. powders, chunks, pellets or granules) of various sizes will enable tailoring of the heat generation profile to provide rapid initial heating to quickly ready the apparatus for use upon actuation, and a sustained period of heating within the target temperature range to produce volatile components for inhalation, to emulate a conventional smoking article which provides a number of puffs over a period of several minutes. The heat generation is to be tailored to the specific heat source chamber 2 provided in the apparatus, as the size and shape of the reaction chamber also affects the heat generation profile.

In certain examples, CaO in different forms and in different amounts may be combined with different volumes of water in different ways in order to produce heat generation profiles with peak or target temperatures in the range 30 to 100°C and exhibiting different periods at those temperatures.

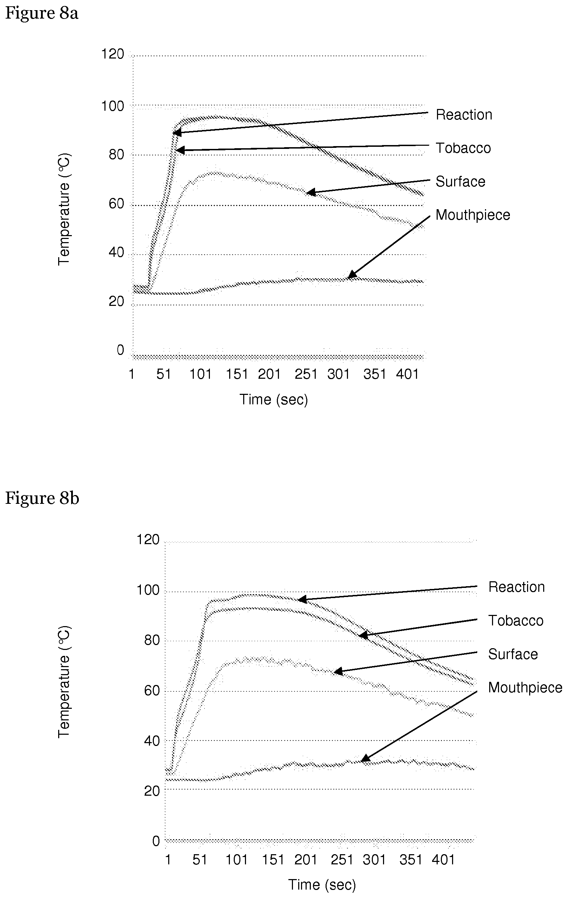

In an attempt to provide an apparatus which will heat a smokeable material to a temperature of at least 80°C within 60 seconds from actuation and maintain a temperature of at least 80°C for at least 300 seconds, the heat source chamber was provided with reactants 4 comprising 1.75 g powdered chunks of CaO having a particle size of approximately 2 mm and 1.25 g of a fine powder of CaO (both forms of CaO obtained from Aldrich Chemicals). The activating reagent used was 2.5 ml of tap water. The smokeable material used was tobacco. The temperature of the heat source, of the tobacco, of the surface of the heating chamber 13, and of the mouthpiece 8 were measured and the results are shown in graphs of Figures 8a and 8b. For the data shown in Figure 8a, the apparatus was held in the vertical orientation, whilst for Figure 8b it was held in the horizontal orientation. The data shows that the orientation had little impact on performance. The temperature of the outer surface of the heating chamber was not equivalent to the surface temperature of a device as it did not include a housing.

Other experiments were conducted to optimise the temperature profile to achieve and maintain a temperature as close to 80°C as possible. The results showed that an exothermic reaction based on 1.75 g of chunks of CaO, 1.25 g of powdered CaO and 3 ml of H₂O produced a good temperature profile for use in the present invention. In an alternative formulation, the exothermic reaction was based on 1.75 g of chunks of CaO, 1 g of powdered CaO and 3ml of H₂O also produced good results.

The effect on the temperature profile of drawing air through the smokeable material was then investigated. The device used to produce the data in Figures 8a and 8b, as described above, was sealed and air was drawn though the heating chamber using a syringe pump to simulate a 35 ml puff. As can be seen from the results set out in the graph of Figure 9, the impact of the air flow on the temperature of the heat source (reaction) and the smokeable material (tobacco) was minimal.

In some alternative examples, the exothermic reaction is an air-based reaction which requires air (specifically oxygen) to initiate the reaction. In one example, the reagent which combines with air to cause an exothermic reaction is iron (Fe). Once again the particle size of the reagent will, to an extent, determine the heat generation profile.

In such examples using an air-based exothermic reaction, the actuating mechanism of the apparatus may involve the opening of vents to allow air to enter the heat source chamber.

In embodiments of the invention, the heat source is a phase change material. A phase change material is a substance which is capable of storing and releasing thermal energy. For example, heat may be released when the material changes from liquid to solid.

In some embodiments, the phase change material is cooled to below the material's freezing temperature without it forming a solid, by a process called supercooling. Once triggered by actuation of the device, such a supercooled fluid will begin to crystallise into its solid phase. Whilst this process is taking place heat will be generated and the temperature of the material will remain at the material's melting temperature.

The generation of heat by phase change material may be self regulating as the rate at which energy leaves the system controls the duration of the phase change. Thus, the temperature of the phase change material remains constant until the material has completely changed from one phase to another. This provides a sustained and constant heat source for heating the smokeable material. The temperature is also predictable and controllable.

Examples of suitable phase change material include, for example, hydrated salts such as sodium acetate trihydrate, sodium hydroxide monohydrate, barium hydroxide octahydrate, magnesium nitrate hexahydrate or magnesium chloride hexahydrate. In some embodiments, the heat source material may further include gelation agents.

The phase change of such materials may be triggered by the introduction of one or more nucleation sites into the phase change material. This may be achieved by homogeneous nucleation, for example, by introducing a seed crystal (a piece of the phase change material in its solid phase) into the liquid phase change material. Alternatively, a small metal tab may be provided within the phase change material which will release a few nuclei (in the form of solid particles of the phase change material which are released from the metal tab surface when it is bent) which can trigger the phase change. In alternative embodiments, the phase change may be triggered by the phase change may be triggered by heterogeneous nucleation. For example, in some embodiments, particles of a different material are added to the liquid phase change material to trigger the phase change. In yet further embodiments, a metal tab may be provided in the phase change material which will work by cavitation, producing microscopic bubbles upon being bent, these bubbles acting as nucleation centres to trigger the phase change.

The rate at which heat energy leaves the heat source chamber and is transferred to the smokeable material in the heating chamber may be controlled by one or more of a number of factors including: the construction of the walls of the heat source chamber and/or the heating chamber, the geometry of the chambers, the materials used and packing density of the smokeable material.

Figure 10 shows an apparatus 21 with a phase change material 24 as the heat source. The phase change material 24 is provided in a heat source chamber 22 which has a tubular configuration and surrounds a heating chamber 33 holding a smokeable material 23. In the illustrated configuration, the heating chamber 13 is cylindrical and is positioned coaxially with the apparatus. In some embodiments, the housing 31 of the apparatus 21 may be insulated, to ensure that the majority of the heat generated by the phase change material 23 is directed internally to the smokeable material 23.

An actuating mechanism 26 is provided which will trigger the phase change reaction upon actuation. In some embodiments, the actuating mechanism involves a sliding element which deforms a metal tab located within the phase change material, thereby releasing nuclei which can trigger the phase change. The heating chamber may be hermetically sealed until the apparatus is ready to deliver the first puff of volatilised components.

In some embodiments, the apparatus 21 may comprise air inlets 35 which allow external air to be drawn into the heating chamber 33 within the housing 31 and through the heated smokeable material 23 during puffing. The path of the airflow is indicated by the arrows in Figure 10. The air inlets 35 may comprise apertures in the housing 31 and may be located upstream from the smokeable material 23 and heating chamber 33 towards the opposite end of the housing 31 to that with the mouthpiece 28. This is shown in Figure 10. Air drawn in through the inlets 35 travels through the heated smokeable material 23 and therein is enriched with smokeable material vapours, such as aroma vapours, before being inhaled by the user at the mouthpiece 28.

Optionally, as shown in Figure 10, the apparatus 21 includes a filter 32 through which the air enriched with smokeable material vapours travels before exiting the apparatus 21 at the mouthpiece 28. Thus, the filter 32 may be positioned between the heating chamber 33 and the mouthpiece 28. The filter 32 may provide physical and/or chemical filtration. In some embodiments, it is provided to prevent particles of the smokeable material from becoming entrained in the air flow and being inhaled. The filter may include a filter material such as cellulose acetate tow, which may be provided in the form of a wrapped plug. Other suitable filters may comprise a wire gauze or the like. Alternatively or in addition, a filter may be included to filter the air being drawn into the apparatus, in which case, the filter optionally may be positioned at the air inlet 35 to filter the air before it enters the heating chamber 33.

Tests using sodium acetate trihydrate showed an increase in temperature of the material from room temperature to 50°C in 2.5-5 seconds upon triggering of the phase change. The temperature was measured in the middle of the fluid in a beaker. The temperature after 8 minutes was measured as being 53.5-54.8°C, with the temperature peaking at a maximum of 54.2-55.2°C.

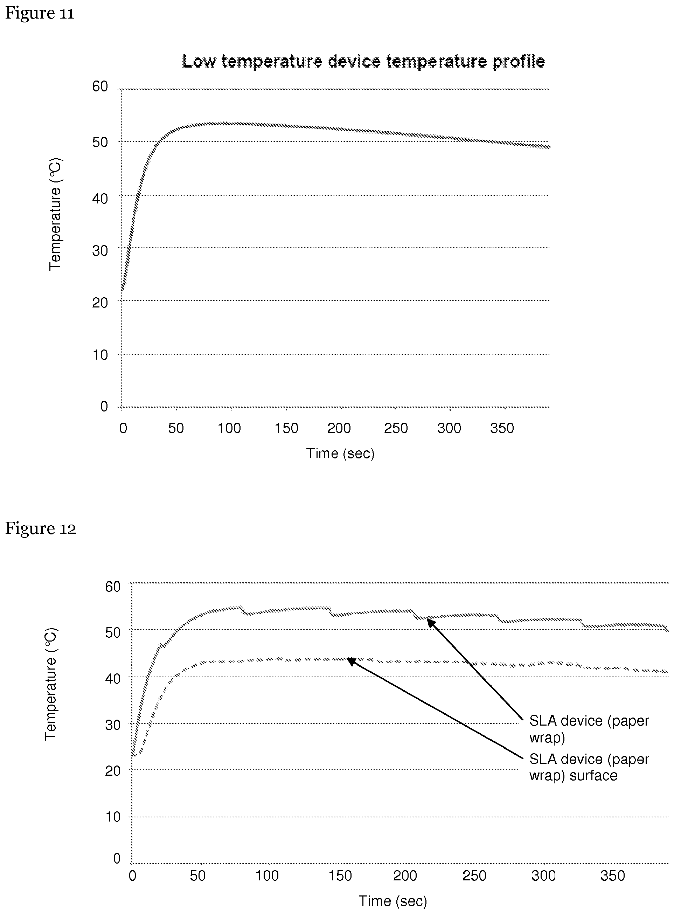

The temperature profile for an apparatus using sodium acetate trihydrate as the heat source is shown in Figure 11. The time taken to reach a target temperature of 50°C was approximately 50 seconds and the heat source remained at or above that temperature for over 300 seconds. The temperature of the outer surface of the apparatus (without a housing) was approximately 45°C.

The effect on the temperature profile of drawing air through the smokeable material was then investigated. The device was sealed and air was drawn though the heating chamber using a syringe pump to simulate a 35 ml puff. As can be seen from the results set out in the graph of Figure 12, the impact of the air flow on the temperature inside the device and at its surface was minimal.

In some configurations of the low or high temperature devices described herein, the smokeable material may be provided in the form of a disposable smokeable material cartridge or other smokeable material consumable which can be inserted into, and removed from, the heating chamber via a suitable opening in the housing. Therefore, a user of the apparatus has the option to replace the smokeable material in order to obtain a different or improved inhalation experience, whilst re-using the housing.

In some embodiments, where the heating chamber is external to the heat source chamber within the apparatus, the apparatus may be provided with an opening which may, for example, comprise a ring-shaped opening located at the housing's end (for example, not at the mouth end) so that the smokeable material cartridge can be slid into the opening and pushed directly into the heating chamber. In other embodiments, the heating chamber is situated centrally within the apparatus and the smokeable material cartridge is cylindrical and can be slid into the apparatus through a central round opening.

In combination with the smokeable material or separately, the heat source may be provided in the form of a removable heat source cartridge which can be inserted into, and removed from, the heat source chamber via a suitable opening in the housing. In the case of heat source which is a phase change material, the heat source may be recharged, for example by heating to effect a change back to the original phase. The user could choose between different heat source cartridges which provide different heat generation profiles, thereby potentially providing different volatilised components for inhalation.

Alternatively, the housing and its internal components, including the smokeable material and the heat source, may together form a disposable item which is intended to be discarded by a user after use.
In some embodiments, the pressure drop of the apparatus may be between about 25 and 200 mmWg and may, for example, be in the region about 50 mmWg. The pressure drop may be controlled by one or more factors including, for example, the density of the packing of the smokeable material, inclusion of filters (positioned before and/or after the smokeable material) and device design, such as including providing apertures of an appropriate size to control airflow in the desired manner.
In some embodiments, the mass of the smokeable material which is heated by the heat source, may be in the range of 0.1 to 1.0 g. The temperature to which the smokeable material is heated may be user controllable, for example to any temperature within the temperature range of 50°C to 150°C. The mass of the apparatus 1 as a whole may be in the range of 2 to 125 g.
It will be appreciated that any of the alternatives described above can be used singly or in combination. This is particularly true of the relative positions of the various parts of the apparatus, including the relative positions of the heat source and the smokeable material to be heated, and the positions of the one or more filters. These may be varied in both the low and high temperature devices described herein.
In order to address various issues and advance the art, the entirety of this disclosure shows by way of illustration various embodiments in which the claimed invention(s) may be practiced and provide for a superior apparatus and method. The advantages and features of the disclosure are of a representative sample of embodiments only, and are not exhaustive and/or exclusive. They are presented only to assist in understanding and teach the claimed features. It is to be understood that advantages, embodiments, examples, functions, features, structures, and/or other aspects of the disclosure are not to be considered limitations on the disclosure and that other embodiments may be utilised, wherein the scope of the invention is defined by the appended claims.

## Claims

1. An apparatus (21) to heat smokeable material (23) to volatilise at least one component of the smokeable material, wherein the apparatus (21) comprises: a housing (31) containing a chemical heat source (24) in a heat source chamber (22) and a heating chamber (33) for containing the smokeable material (23), wherein the chemical heat source (24) comprises a phase change material (24); a mouthpiece (28) in fluid communication with the heating chamber (33); and an actuating mechanism (26), wherein the actuating mechanism (26), upon actuation, activates the phase change material (24) to generate heat which heats the smokeable material (23); and **characterised in that** at least some of the phase change material (24) is visible from the outside of the apparatus (21).

2. An apparatus (21) as claimed claim 1, wherein the apparatus (21) further comprises the smokeable material (23) to be heated so that at least one component of said smokeable material (23) is volatilised.

3. An apparatus (21) as claimed in claim 1 or claim 2, wherein the heat source (24) and/or the smokeable material (23) is heated to a target temperature of 80-125°C.

4. An apparatus (21) as claimed in claim 1 or claim 2, wherein the heat source (24) and/or the smokeable material (23) is heated to a target temperature of 40-80°C.

5. An apparatus (21) as claimed in claim 3 or claim 4, wherein the target temperature is reached within no more than 5 minutes from actuation of the actuating mechanism (26).

6. An apparatus (21) as claimed in any one of claims 3 to 5, wherein the target temperature is maintained for a period of at least 3 minutes.

7. An apparatus (21) as claimed in any preceding claim, further comprising a filter (32).

8. An apparatus (21) as claimed in any preceding claim, wherein the phase change material (24) is selected from the group consisting of: sodium acetate trihydrate, sodium hydroxide monohydrate, barium hydroxide octahydrate, magnesium nitrate hexahydrate and magnesium chloride hexahydrate.

9. An apparatus (21) as claimed in any preceding claim, wherein at least a portion of the housing (33) is clear or translucent.

10. An apparatus (21) as claimed in any preceding claim, wherein at least a portion of a wall of the heat source chamber (22) is clear or translucent.

11. An apparatus (21) as claimed in any preceding claim, wherein the heat source chamber (22) at least partially surrounds the heating chamber (33).

12. An apparatus (21) as claimed in any preceding claim wherein the actuating mechanism (26) comprises one of: a seed crystal for introducing into the phase change material (24); a small metal tab within the phase change material (24) for releasing solid particles of the phase change material (24) when it is bent to trigger the phase change; particles of a different material to the phase change material (24) which are added to the phase change material (24) to trigger the phase change; a metal tab provided in the phase change material (24) for producing microscopic bubbles upon being bent which act as nucleation sites to trigger the phase change; a sharp point or clicker for providing a nucleation site.

13. An apparatus (21) according to any preceding claim wherein the chemical heat source (24) is provided in the form of a removable heat source cartridge which can be inserted into and removed from the heat source chamber (22) via an opening in the housing (31).

## Patentansprüche

1. Vorrichtung (21) zum Erwärmen von rauchbarem Material (23), um mindestens eine Komponente des rauchbaren Materials zu verflüchtigen, wobei die Vorrichtung (21) umfasst: ein Gehäuse (31), welches eine chemische Wärmequelle (24) in einer Wärmequellkammer (22) enthält, und eine Heizkammer (33), um das rauchbare Material (23) zu enthalten, wobei die chemische Wärmequelle (24) ein Phasenänderungsmaterial (24) umfasst; ein Mundstück (28) in fließtechnischer Kommunikation mit der Heizkammer (33); und einen Betätigungsmechanismus (26), wobei der Betätigungsmechanismus (26) bei Betätigung das Phasenänderungsmaterial (24) aktiviert, um Wärme zu generieren, die das rauchbare Material (23) erwärmt; und **dadurch gekennzeichnet, dass** mindestens ein Teil des Phasenänderungsmaterials (24) von außerhalb der Vorrichtung (21) sichtbar ist.

2. Vorrichtung (21) nach Anspruch 1, wobei die Vorrichtung (21) ferner umfasst, dass das rauchbare Material (23) so erwärmt wird, dass mindestens eine Komponente des rauchbaren Materials (23) verdampft wird.

3. Vorrichtung (21) nach Anspruch 1 oder Anspruch 2, wobei die Wärmequelle (24) und/oder das rauchbare Material (23) auf eine Zieltemperatur von 80 bis 125 °C erwärmt wird bzw. werden.

4. Vorrichtung (21) nach Anspruch 1 oder Anspruch 2, wobei die Wärmequelle (24) und/oder das rauchbare Material (23) auf eine Zieltemperatur von 40 bis 80 °C erwärmt wird bzw. werden.

5. Vorrichtung (21) nach Anspruch 3 oder Anspruch 4, wobei die Zieltemperatur in nicht mehr als 5 Minuten ab Betätigung des Betätigungsmechanismus (26) erreicht wird.

6. Vorrichtung (21) nach einem der Ansprüche 3 bis 5, wobei die Zieltemperatur für einen Zeitraum von mindestens 3 Minuten gehalten wird.

7. Vorrichtung (21) nach einem der vorhergehenden Ansprüche, ferner umfassend einen Filter (32).

8. Vorrichtung (21) nach einem der vorhergehenden Ansprüche, wobei das Phasenänderungsmaterial (24) ausgewählt ist aus der Gruppe bestehend aus Natriumacetat-Trihydrat, Natriumhydroxid-Monohydrat, Bariumhydroxid-Octahydrat, Magnesiumnitrat-Hexahydrat und Magnesiumchlorid-Hexahydrat.

9. Vorrichtung (21) nach einem der vorhergehenden Ansprüche, wobei mindestens ein Anteil des Gehäuses (33) klar oder durchscheinend ist.

10. Vorrichtung (21) nach einem der vorhergehenden Ansprüche, wobei mindestens ein Anteil einer Wand der Wärmequellkammer (22) klar oder durchscheinend ist.

11. Vorrichtung (21) nach einem der vorhergehenden Ansprüche, wobei die Wärmequellkammer (22) die Heizkammer (33) mindestens teilweise umgibt.

12. Vorrichtung (21) nach einem der vorhergehenden Ansprüche, wobei der Betätigungsmechanismus (26) eines der folgenden umfasst: einen Impfkristall zum Einbringen in das Phasenänderungsmaterial (24); eine kleine Metalllasche innerhalb des Phasenänderungsmaterials (24), um feste Partikel des Phasenänderungsmaterials (24) freizusetzen, wenn sie gebogen wird, um die Phasenänderung auszulösen; Partikel eines anderen Materials als das Phasenänderungsmaterial (24), die dem Phasenänderungsmaterial (24) zugefügt werden, um die Phasenänderung auszulösen; eine Metalllasche, die in dem Phasenänderungsmaterial (24) bereitgestellt wird, um mikroskopische Bläschen zu erzeugen, wenn sie gebogen wird, die als Kristallkeimstellen wirken, um die Phasenänderung auszulösen; eine Zacke oder einen Klicker, um eine Kristallkeimbildungsstelle bereitzustellen.

13. Vorrichtung (21) nach einem der vorhergehenden Ansprüche, wobei die chemische Wärmequelle (24) in Form einer entfernbaren Wärmequellkartusche bereitgestellt wird, die über eine Öffnung in dem Gehäuse (31) in die Wärmequellkammer (22) eingesetzt und entfernt werden kann.

## Revendications

1. Appareil (21) pour chauffer un matériau à fumer (23) pour volatiliser au moins un composant du matériau à fumer, l'appareil (21) comprenant : un boîtier (31) contenant une source de chaleur chimique (24) dans une chambre de source de chaleur (22) et une chambre de chauffage (33) pour contenir le matériau à fumer (23), dans lequel la source de chaleur chimique (24) comprend un matériau à changement de phase (24) ; un embout (28) en communication fluidique avec la chambre de chauffage (33) ; et un mécanisme d'actionnement (26), dans lequel le mécanisme d'actionnement (26), lors de l'actionnement, active le matériau à changement de phase (24) pour générer de la chaleur qui chauffe le matériau à fumer (23) ; et **caractérisé en ce qu'**au moins une partie du matériau à changement de phase (24) est visible depuis l'extérieur de l'appareil (21).

2. Appareil (21) selon revendication 1, l'appareil (21) comprenant en outre le matériau à fumer (23) devant être chauffé de sorte qu'au moins un composant dudit matériau à fumer (23) soit volatilisé.

3. Appareil (21) selon la revendication 1 ou la revendication 2, dans lequel la source de chaleur (24) et/ou le matériau à fumer (23) est chauffé à une température cible de 80 à 125 °C.

4. Appareil (21) selon la revendication 1 ou la revendication 2, dans lequel la source de chaleur (24) et/ou le matériau à fumer (23) est chauffé à une température cible de 40 à 80 °C.

5. Appareil (21) selon la revendication 3 ou la revendication 4, dans lequel la température cible est atteinte en pas plus de 5 minutes à partir de l'actionnement du mécanisme d'actionnement (26).

6. Appareil (21) selon l'une quelconque des revendications 3 à 5, dans lequel la température cible est maintenue pendant une durée d'au moins 3 minutes.

7. Appareil (21) selon l'une quelconque des revendications précédentes, comprenant en outre un filtre (32) .

8. Appareil (21) selon l'une quelconque des revendications précédentes, dans lequel le matériau à changement de phase (24) est choisi dans le groupe constitué de : l'acétate de sodium trihydraté, l'hydroxyde de sodium monohydraté, l'hydroxyde de baryum octahydraté, le nitrate de magnésium hexahydraté et le chlorure de magnésium hexahydraté.

9. Appareil (21) selon l'une quelconque des revendications précédentes, dans lequel au moins une partie du boîtier (33) est transparente ou translucide.

10. Appareil (21) selon l'une quelconque des revendications précédentes, dans lequel au moins une partie d'une paroi de la chambre de source de chaleur (22) est transparente ou translucide.

11. Appareil (21) selon l'une quelconque des revendications précédentes, dans lequel la chambre de source de chaleur (22) entoure au moins partiellement la chambre de chauffage (33).

12. Appareil (21) selon l'une quelconque des revendications précédentes, dans lequel le mécanisme d'actionnement (26) comprend l'un parmi : un germe cristallin pour introduction dans le matériau à changement de phase (24) ; une petite languette métallique dans le matériau à changement de phase (24) pour libérer des particules solides du matériau à changement de phase (24) lorsqu'elle est pliée pour déclencher le changement de phase ; des particules d'un matériau différent du matériau à changement de phase (24) qui sont ajoutées au matériau à changement de phase (24) pour déclencher le changement de phase ; une languette métallique disposée dans le matériau à changement de phase (24) pour produire des bulles microscopiques lorsqu'elle est pliée, qui agissent en tant que sites de nucléation pour déclencher le changement de phase ; une pointe tranchante ou un coupeur pour produire un site de nucléation.

13. Appareil (21) selon l'une quelconque des revendications précédentes, dans lequel la source de chaleur chimique (24) est fournie sous la forme d'une cartouche de source de chaleur amovible qui peut être insérée dans et retirée de la chambre de source de chaleur (22) par l'intermédiaire d'une ouverture dans le boîtier (31) .
